# EUROPEAN PATENT SPECIFICATION

(11) **EP 1 842 516 B1**
(45) Date of publication and mention of the grant of the patent: **11.11.2009**
(21) Application number: 06425238.0
(22) Date of filing: 05.04.2006
(51) Int. Cl.: A61F 13/494

(54) **A method and device for producing elasticated sanitary products**
Verfahren und Vorrichtung zum Produzieren van elastischen Hygieneartikeln
Procédé et appareil pour fabriquer des articles hygiéniques élastiques

(43) Date of publication of application: 10.10.2007
(73) Proprietor: Fameccanica.Data S.p.A., 66020 Sambuceto di S. Giovanni Teatino (Chieti) (IT)
(72) Inventor: Giansante, Antonio, 65010 Spoltore (Pescara) (IT); Polidori, Domenico, 66010 Tollo (Chieti) (IT); Simone, Giambattista, 65010 Spoltore (Pascara) (IT); Gagliardone, Paolo, 65020 Alanno (Pescara) (IT)
(74) Representative: Bosotti, Luciano

(56) References cited:
- EP-A- 1 563 817

## Description

### Field of the invention

The invention relates to techniques for manufacturing elasticated sanitary products, that is sanitary products including a material bearing at least one elastic element applied onto it. The invention was developed with particular attention to its possible use in manufacturing absorbent sanitary products that can be worn in the guise of a panty.

### Description of the known technology

In the production of sanitary products that can be worn in the guise of panties (for example diapers or trainer pants for babies or incontinence pads) it is the usual practice to apply elastic elements onto the product destined to favour better adherence of the product to the wearer's body, in particular with regard to the perineal region of the wearer.

In general, and in particular with regard to diapers and trainer pants for babies, these elastic elements comprise:
- "transversal" elastic strips applied in correspondence with portions of the product destined, when the product is worn, to define the waistline of the product, and
- "longitudinal" elastic strips applied at the sides of the so-called "crotch portion" of the product so that the parts of the product destined to extend around the thighs of the wearer, when the product is worn, embrace the wearer's body in a sufficiently close fashion, contrasting the undesired dispersion of bodily excretions.

The techniques used by almost all manufacturers entail that the sanitary product in question is obtained by segmenting a chain or web of products in movement -- usually at rather fast speedssubstantially similar to a belt.

Two basic philosophies underlie these manufacturing techniques, that is:
-- "machine direction" techniques, in which the products comprised in the chain or web advance "lengthways", that is with the part that will be the anterior extremity of one product connected to the posterior extremity of the adjacent product in the chain, and
-- "cross direction" techniques, in which the individual products are oriented "crosswise" with regard to the chain, and are thus connected one to the next, before the segmentation that will lead to the formation of individual products, in correspondence with their lateral margins.

Whichever solution is adopted (machine direction or cross direction) the elastic strips are applied either transversely with regard to the direction of advancement of the chain of products, or in the direction of advancement of such chains.

More specifically the invention relates to a device according to claim 1, which is known, e.g. from EP-A-1 563 817.

### Object and summary of the invention

Above all in the manufacture of incontinence pads, chiefly destined for an adult consumer and thus such as to present relatively large dimensions, the need is felt to apply elastic elements with more marked "anatomical" character, that is elastic elements that more closely follow the anatomical shape of the pad when worn. This must also take into account that the elastic elements applied onto the incontinence pads exercise a relatively strong force of elastic return. This means that relatively strong elastic must be used and brings with it the additional difficulty of ensuring a solid connection between the elastic and the product onto which it is applied; the final operations of folding the product in view of its packaging indeed require that the product remain sufficiently flat, avoiding undesired phenomena of wrinkling induced by the presence of the elastic elements.

The object of the invention is to meet the above described needs in an ideal fashion.

According to the present invention, this object is achieved thanks to a device having the characteristics described specifically in Claim 1.

The claims form an integral part of the disclosure of the invention provided herein.

Those of skill in the art will appreciate that, although developed with specific attention to its possible use in manufacturing incontinence pads, the solution described here lends itself to be used advantageously also to produce sanitary products of different types.

### Short description of the attached representations

The invention will now be described, as a pure example without limiting intent, with reference to the attached drawings, in which:
- figure 1 is a general diagram demonstrating the principle underlying the solution described herein,
- figure 2 is a general perspective view of a device of the type described here,
- figure 3 is a perspective view of one of the elements of the device in figure 2, represented alone, and
- figure 4 is an enlarged view of the part of figure 3 indicated by arrow IV.

### Detailed description of some embodiments of the invention

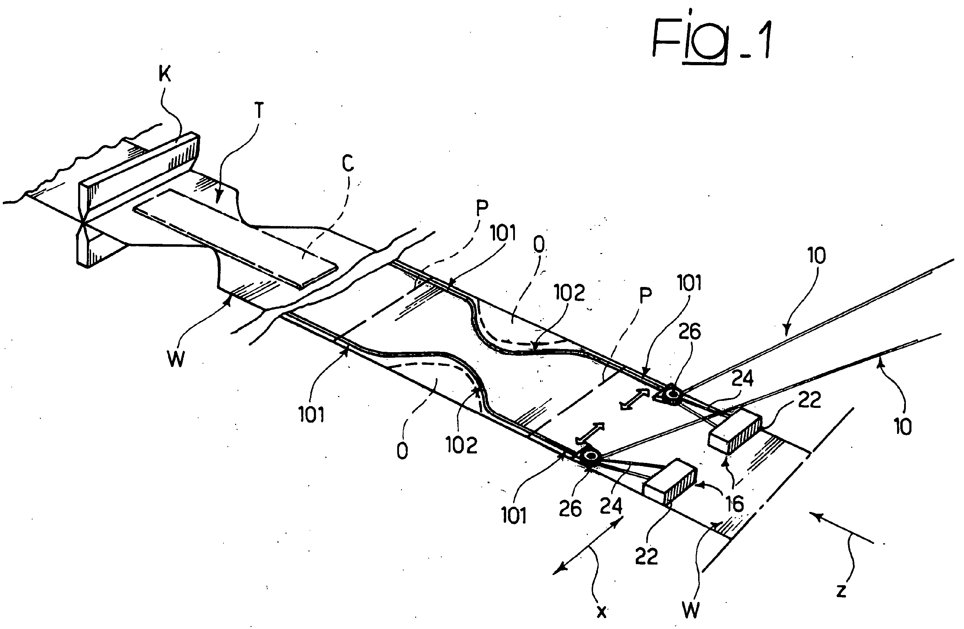

In the general diagram in figure 1, reference W indicates a material in web form advancing in a direction z. In the specific case, the material W may be one of the sheets used to produce a sanitary product such as for example an incontinence pad. For example, the material W may be constituted of a sheet of plastic material (e.g. polyethylene) impermeable to liquids destined to constitute the external sheet or "backsheet" of the product.

In essential terms, the production cycle of the product entails that absorbent elements (cores) C are superimposed, at regular intervals, onto the backsheet W, followed by an upper layer, produced starting from a web material form, in general permeable to liquids and destined to constitute the so-called "topsheet" T of the product. As is known, by topsheet is meant the sheet destined to be positioned towards the wearer's body so as to collect the bodily liquids that, passing through the topsheet T, are collected in the absorbent core C.

The composite web thus obtained is then subjected to segmentation (see the cutting station diagrammatically indicated with K in figure 1) so as to obtain the individual products.

All this corresponds to criteria well known in the art that do not require a detailed description here. Equally known is the fact that numerous other accessory elements may be included in the overall structure of the sanitary product.

These production details of the product overall are not of specific significance for the purpose of understanding the present invention, which is of itself capable of being applied to a wide range of different types of product.

Specifically, the solution described here as an example tackles the problem of applying to the sheet W (here indicated as an example as a backsheet) "anatomic" elastic elements overall indicated with 10.

With reference to the individual product (whose outer margins are indicated in diagram form with the dotted line P in figure 1) the elastic elements 10 essentially consist of two homologous elements, that jointly follow - in whole or in part - the final hourglass profile given to the product (this too following well-known criteria) so as to form openings O for the passage of the legs of the person wearing the product in the guise of a panty.

To achieve this result, the elastic elements 10 are applied onto the web W following a pathway that may be defined as an S-shaped or serpentine pathway. In detail, this pathway comprises an alternation of straight stretches 101 and generically arched portions 102 that extend inwards, that is towards the central longitudinal axis, of the web material W.

Formation of the lateral openings O may precede or follow application of the elastic elements 10. Again, it will be appreciated that -- for the purposes of the present description -- the web W may in practice be considered as representative of a chain of sanitary products undergoing formation. This despite the fact, in rigorous terms, the web W does not yet of itself constitute a complete sanitary product.

As may be deduced from observing figure 1, two elongated elastic elements 10 are applied onto the web W, one for each side of the web W, that follow symmetrical mirror-image trajectories with regard to the longitudinal median axis of the web W. In the remainder of this description, almost exclusive reference will thus be made to the application of one of said elements 10, being it otherwise understood that these criteria -- with the exception of their mirror-image arrangement -- also apply to the other element 10 taken into consideration.

The elements 10 may comprise a single filiform elastic element, for example a rubber-based strip or thread, of the type generally used to elasticate sanitary products. Alternatively, the elements 10 may in reality comprise more than one elastic formation (for example a number of elastic threads coupled together). In the embodiment illustrated here, which is only an example, it will be supposed that each element 10 is constituted of a limited number (3-4) of elastic threads destined to be positioned one alongside the others.

The thread or threads of each element 10 unwind starting from a source of supply S (not illustrated in detail, but of known type) consisting of a delivery unit 12 that usually comprises a number of grooves 14 through which the individual elasticated threads pass.

To each of the grooves 14 a respective control device may be associated to control the speed at which the elastic thread runs destined to operate such that, in the passage between the delivery unit 12 and the web W the elastic elements 10 are subjected to a force of traction in exactly controlled conditions. This so as to precisely regulate the force of elastic return that the elements 10 will exercise once applied onto the web W.

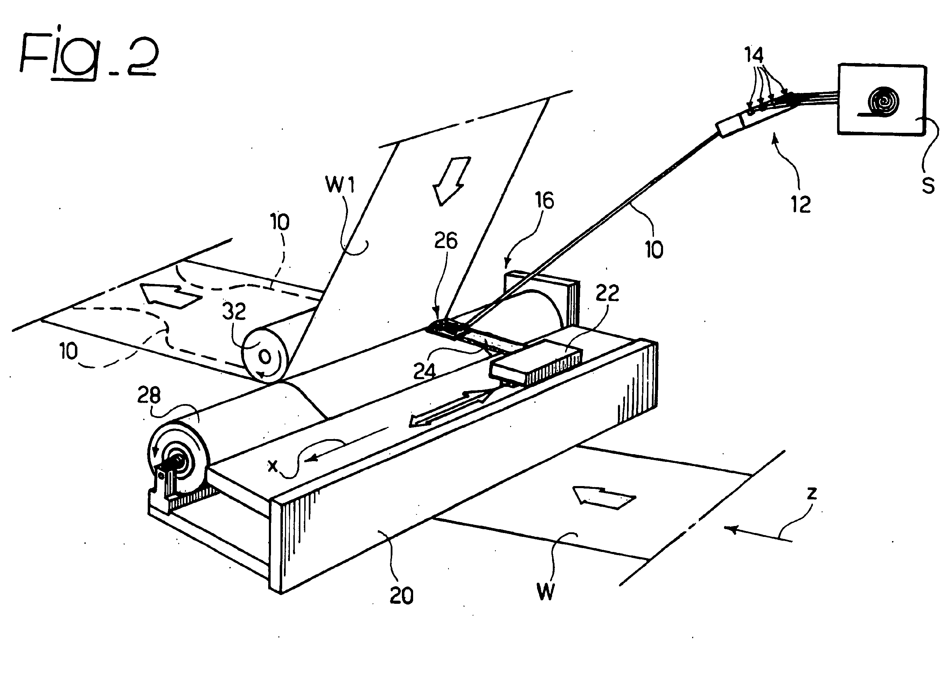

The transfer movement of the elastic element 10 (henceforth it will be considered as a single element, although in reality it comprises an array of parallel elastic threads) towards the web W, is guided -- on each side of the web W -- by a guide unit or deflector unit indicated overall with 16. For simplicity of illustration, only one of these units is illustrated in figure 2.

The unit (or each unit) 16 is located on a support structure 20 and includes a slide 22, mounted such that it is selectively mobile on the structure 20 along an axis x orthogonal to the axis z of advancement of the web W. The slide 22 bears projecting forwards an arm 24, on whose distal extremity is mounted a revolving head to guide delivery 26.

As will be better understood below, the elements 20, 22 and 24 form a structure to impart movement that selectively displaces the "applicator" head 26 along the axis x. Each slide 22 usually comprises a body of small size and low inertia (that can be made either of a light metal material or of a composite material including for example carbon fibres) capable of moving back and forth along the axis x under the action of a motor element (not clearly visible in the drawings, but of known type). All of this such as to cause the arm 24 and, more to the purpose, the delivery head 26 mounted at the distal extremity of the arm 24 to move in correspondence with the surface of the web W along the axis x.

Given that -- at least in the exemplary embodiment illustrated here -- the elastic elements 10 to be applied are two in number (one on each side of the web W), it is understood that on the structure 20 in reality two slides 22 are mounted bearing respective arms 24 each having at its distal extremity a delivery head 26. In view of the symmetrical placement of the elastic elements 10, the movement of the slides 22 is commanded, through their respective motors, in a symmetrical fashion with regard to the axis x.

In consideration of this substantial symmetry, the description will now be continued with reference to the description of the structure of a single slide 22 and of the parts associated to it.

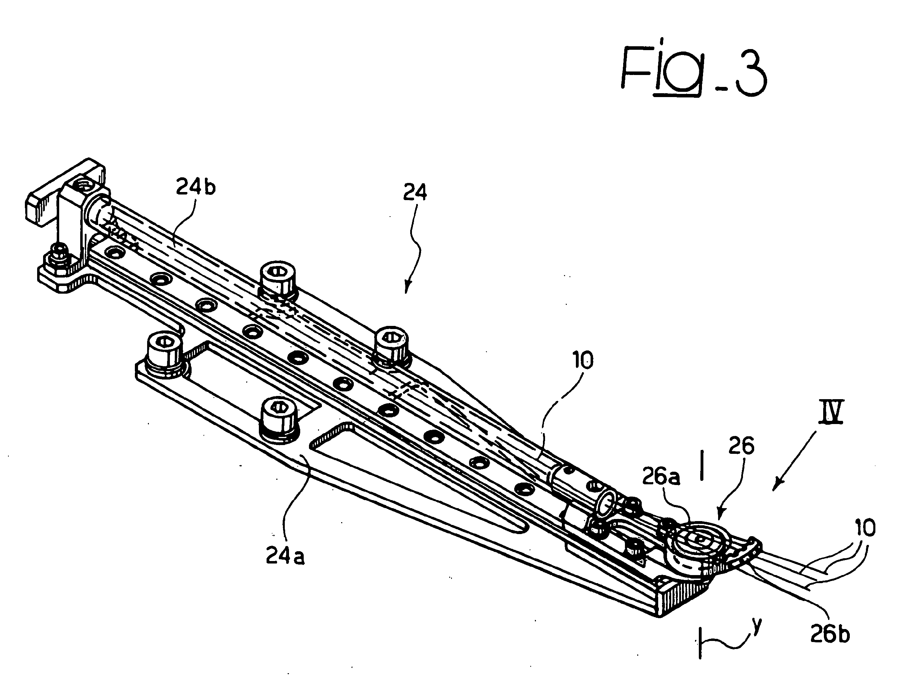

As is more clearly illustrated in figure 3, the arm 24 comprises a supporting plate 24a of light material (for example light metal alloy and/or having an aperturated structure) that projects starting from the slide 22 slightly downwards such as to bring its distal extremity where the head 26 is mounted. The structure 24a supports a tubular element 24b that acts as a guide for the passage of the elastic element 10 towards the delivery head 26.

The head 26 is destined to be located in correspondence with the plane on which the web W runs. As is illustrated in figure 2, the web W in general passes below the structure 20 after which it is wound onto a return roller 28 situated in correspondence with the zone to which the elastic element 10 is guided by the head 26 and brought into contact with the upper face of the web W.

The roller 28 acts (together with other rollers not visible in the drawings) as a drive element that causes the web W to advance in the direction of advancement, identified by the axis z.

By exploiting the combination of the advancement movement of the web material W in the direction z (from right to left, with reference to the point of observation of figures 1 and 2) and of the transverse motion according to the axis x capable of being given to each of the slides 22 (and thus to the arms 24 and to the respective delivery heads 26 borne by them) it is possible to give virtually any desired application trajectory to the portion of the elastic element 10 brought at a specific moment in time into proximity with the web W, that is any trajectory capable of being obtained by effect of superimposing the two movements: i) of the web W (and of the elastic element 10 applied onto it) and ii) of each of the slides 22.

In particular, should it be desired to achieve the application trajectory mentioned above (straight stretches 101 alternated with "half-waves" 102 projecting towards the central longitudinal axis of the web W) it is possible to operate as follows:
- hold the slides 22 stationary in a generically lateral position with regard to the web W during the phase of application of the straight stretches 101, and
- move the slides 22 towards the central longitudinal axis of the web W to achieve the concave zones 102.

Naturally, this result may be achieved since, once applied onto the web material W, the material constituting the portion or stretch of elastic element at that moment being applied onto the web W is anchored onto the web W so as not to move further with regard to this.

This result may be achieved in different ways.

A first solution is that of fabricating the element 10 (and the parts that make it up) with an adhesive material that, when brought into contact with the web W, remains anchored to the surface of the same.

Another solution (at present preferred and thus represented in greater detail in figure 2) entails that in correspondence with the roller 28, immediately downstream from the zone where elastic element 10 is brought into contact with the web W (this is a linear zone parallel to the x axis), a "counter-web" W1 of homologous or similar material is bonded to the web W. The counter-web W1 is pressed against the web material W such that the web W and the counter-web W1 (with the element 10 interposed between them) are made to pass through a pair of rollers comprising a roller 28 and a counter-roller 32.

This solution has the result that, once the elastic element 10 is applied onto the web W, it becomes "trapped" or sandwiched between the webs or sheets W and W1.

This solution may be used in combination with the fact of applying the elastic element 10 in an adhesive fashion onto the sheet W (or, in an asymmetric fashion, onto the web W1), or simply by exploiting the action of the mechanical trapping of the elastic material between the sheets W. This trapping effect -- aimed at avoiding the lateral sliding of the element 10 with regard to the web W -- may to advantage be achieved by providing for a sufficiently close connection between the sheets W and W1 obtained, for example, through adhesive bonding, heat welding, etc.

With reference to the embodiment at present preferred, and illustrated here, the fact that the elastic elements 10 are sandwiched between the sheets W and W1 means that the elastic elements 10 can no longer move with respect to the web W. Furthermore, the sandwiched condition described contrasts the phenomena of wrinkling that might be induced by the action of elastic return exercised by the elements 10. It will be appreciated in particular that, adopting this fixing modality for the elastic elements 10 onto the web W, that is application of a counter-web W1, no traces of adhesive will remain on the surface of the web W if an adhesive is used to fix the elastic elements 10.

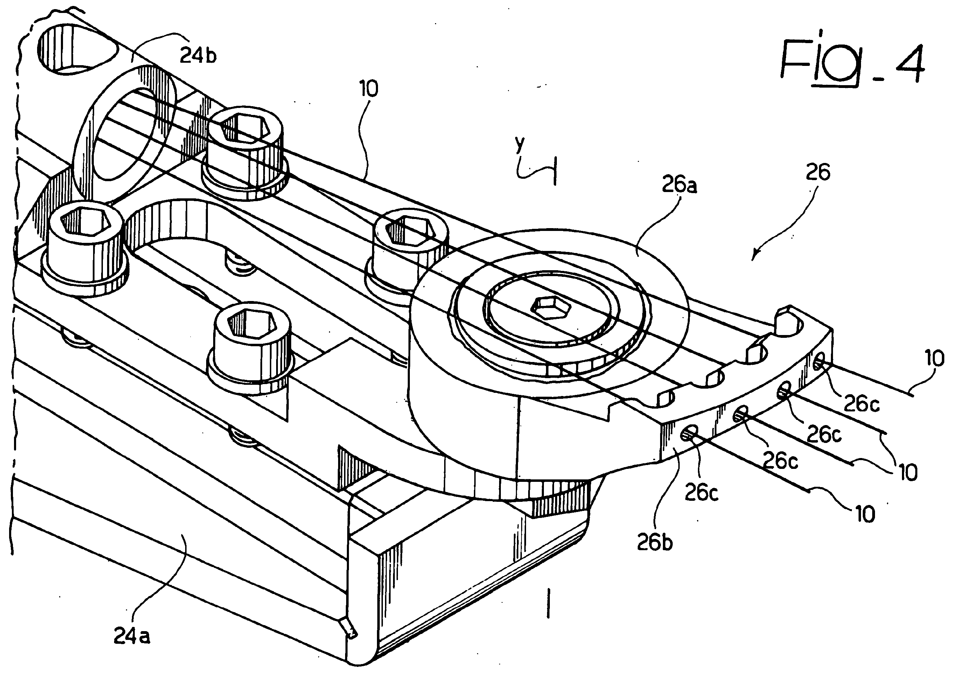

As can better be understood in the view in figure 4, the revolving head 26 for preference takes the form of a sort of small hand comprising a "carpal" portion 26a mounted at the distal extremity of the arm 24 free to rotate around an axis y substantially orthogonal with regard to the plane defined jointly by the axes z (advancement of the web W) and x (transverse movement of the slides 22).

Usually, as well as said ability to rotate, the portion 26a can also pivot in an angular manner with regard to the axis y due to the play in its mounting onto the distal extremity of the arm 24.

From the portion 26a a plurality of "fingers" 26b extend - each of which bears an opening 26c (typically this is a hole). The openings 26c are destined to act as guide formations for a respective elongated element (for example a thread) included in the elastic element 10 that, arriving from the tube 24b, passes through one of the holes 26c before being applied onto the web W.

The ability to revolve and pivot with respect to the y axis described above means that the openings 30 are constantly oriented according to the direction of the part of elastic element 10 that at that particular moment is being applied onto the web W.

For obvious considerations of a geometric nature, this corresponds to a situation such that, at the moment in which it is applied against the web W, the elastic element 10 has no appreciable lateral translation movement with respect to the material of the web W.

It will also be appreciated that, by making the arms 24 of the two slides 22 of different lengths and by causing them to extend according to trajectories (if necessary elbow-shaped) that do not interfere with one another, it is also possible to impart to the respective delivery heads 26 relative movements in the sense of a superimposition and/or an alternation of their positions such as to lead to crossing over of the trajectories of the two elements 10 deposited onto the web element W.

Again, it will be appreciated that the criteria described above may also be extended to any number of slide 22 - arm 24 - delivery head 26 units, it thus being possible to provide for both solutions requiring a single unit of this type (which applies a single elastic element onto the web W) and solutions that require three or more of these applicator units with the consequent possibility of applying onto the web W three or more elastic elements 10. All of this while being able, in the fashion described above, to adjust (if appropriate in a differentiated manner for each of the elastic elements 10) the condition of traction applied to the individual elastic element.

It follows that, without prejudice to the underlying principle of the invention, the construction details and embodiments may be widely varied with regard to what is described and illustrated, without thereby departing from the extent of protection as determined by the attached claims.

## Claims

1. A device to fabricate sanitary products comprising a material (W) having applied thereto at least one elastic element (10), the device including:
-- at least one drive element (28) to cause a web (W) of said material to advance in a given direction of advancement (z),
-- a source (12, 14) of delivery of at least one elongated elastic element (10),
-- an applicator head (26) to apply onto said web material (W) said at least one elongated elastic element (10), and
-- a support structure (20, 22, 24) to move said applicator head (26) selectively in a transverse direction (x) with respect to said direction of advancement (z) of said web material (W), imparting to said elastic material (10) applied onto said web material (W) a trajectory with loops, wherein said supporting structure (20, 22, 24) comprises a slide element (22) bearing said applicator head (26), said slide element (22) being mobile in the transverse direction (x) with respect to said direction of advancement (z) and bearing said applicator head (26) by means of an arm (24) projecting from said slide element (22) and bearing at the distal extremity said applicator head (26),
**characterised in that** said applicator head (26) is a revolving head with a hand-like configuration with a carpal portion (26a) and a plurality of fingers (26b) bearing respective guide openings (26c) for said elastic formations comprising said at least one elongated elastic element (10), wherein said applicator head (26) is rotatable around an axis (y) substantially orthogonal to said direction of advancement (z) of said web material (W) and to said transverse direction (x) with respect to said direction of advancement (z) of said web material (W) so as to follow the orientation movement of said at least one elongated elastic element (10) during its application onto said web material.

2. The device according to claim 1, **characterised in that** it includes an applicator element (32) for applying onto said web material (W), in correspondence with said applicator head (26) a further web material (W1) to enclose, according to a general sandwich configuration, said at least one elongated elastic element (10) between said web material (W) and said further web material (W1).

3. The device according to either of the preceding claims 1 or 2, **characterised in that** it includes control elements of the delivery (12, 14) of said at least one elongated elastic element (10) in the portion between said source of supply (12) and said applicator head (26).

4. The device according to any of the preceding claims 1 to 3, **characterised in that** said applicator head (26) comprises a series of guide openings (26c) to guide a respective series of elastic formations constituting said at least one elongated elastic element (10).

5. The device according to any of the preceding claims 1 to 4, **characterised in that** it includes:
- at least two of said sources (12) delivering elongated elastic elements (10),
- at least two said applicator heads (26) to apply onto said web material (W) at least two elongated elastic elements (10).

## Patentansprüche

1. Vorrichtung zur Herstellung von Sanitärprodukten, ein Material (W) umfassend, an welchem zumindest ein elastisches Element (10) angebracht ist, wobei die Vorrichtung umfasst:
- zumindest ein Antriebselement (28), um ein Netz (W) des Materials in einer vorgegebenen Fortbewegungsrichtung (z) fortzubewegen,
- eine Quelle (12, 14) der Lieferung von zumindest einem länglichen elastischen Element (10),
- ein Applikatorkopf (26) zum Anbringen auf dem Netzmaterial (W) des zumindest einen länglichen elastischen Elements (10), und
- eine Stützstruktur (20, 22, 24) um den Applikatorkopf (26) selektiv in eine Querrichtung (x) in Bezug auf die Beförderungsrichtung (z) des Netzmaterials (W) zu verschieben, und um dem elastischen Material (10), welches an dem Netzmaterial (W) angebracht ist, eine Trajektorie mit Schlaufen zu verleihen, wobei die Stützstruktur (20, 22, 24) ein Gleitelement (22) umfasst, welches den Applikatorkopf (26) stützt, wobei das Gleitelement (22) in Querrichtung (x) in Bezug auf die Beförderungsrichtung (z) verschiebbar ist und den Applikatorkopf (26) mittels eines Arms (24) trägt, welches von dem Gleitelement (22) heraussteht und am distalen Ende den Applikatorkopf (26) stützt,
**dadurch gekennzeichnet, dass** der Applikatorkopf (26) ein Drehkopf mit einer handartigen Konfiguration mit einem Handwurzelknochen-Abschnitt (26a) und einer Vielzahl von Fingern (26b) ist, welche entsprechende Führungsöffnungen (26c) für die elastischen Formen tragen, welche das zumindest eine längliche elastische Element (10) umfassen, wobei der Applikatorkopf (26) drehbar um eine Achse (y) ist, welche im Wesentlichen orthogonal zu der Beförderungsrichtung (z) des Netzmaterials (W) und zu der Querrichtung (x) in Bezug auf die Beförderungsrichtung (z) des Netzmaterials (W) ist, um der Orientierungsverschiebung des zumindest einen länglichen elastischen Elements (10) während seiner Anbringung an dem Netzmaterial zu folgen.

2. Vorrichtung nach Anspruch 1, **dadurch gekennzeichnet, dass** sie ein Applikatorelement (32) zum Anbringen an dem Netzmaterial (W) umfasst und in Übereinstimmung mit dem Applikatorkopf (26) ein weiteres Netzmaterial (W1) umfasst, um das zumindest eine längliche elastische Element gemäß einer allgemeinen Sandwich-Konfiguration zwischen dem Netzmaterial (W) und dem weiteren Netzmaterial (W1) einzuschließen.

3. Vorrichtung nach einem der vorhergehenden Ansprüche 1 oder 2, **dadurch gekennzeichnet, dass** er Steuerelemente für die Lieferung (12, 14) des zumindest einen länglichen elastischen Elements (10) in dem Abschnitt zwischen der Versorgungsquelle (12) und dem Applikatorkopf (26) umfasst.

4. Vorrichtung nach einem der vorhergehenden Ansprüche 1 bis 3, **dadurch gekennzeichnet, dass** der Applikatorkopf (26) eine Reihe von Führungsöffnungen (26c) umfasst, um eine jeweilige Reihe von elastischen Formen zu führen, welche das zumindest eine längliche elastische Element (10) ausmachen.

5. Vorrichtung nach einem der vorhergehenden Ansprüche 1 bis 4, **dadurch gekennzeichnet, dass** sie umfasst:
- zumindest zwei der Quellen (12), welche verlängerte elastische Elemente (10) liefern,
- zumindest zwei der Applikatorköpfe (26), um auf das Netzmaterial (W) an zumindest zwei verlängerten elastischen Elementen (10) anzubringen.

## Revendications

1. Un dispositif pour fabriquer des produits sanitaires comprenant un matériau (W) sur lequel a été appliqué au moins un élément élastique (10), le dispositif comprenant :
- au moins un élément d'entraînement (28) pour faire avancer une nappe (W) dudit matériau dans une direction donnée d'avance (z),
- une source (12, 14) de délivrance d'au moins un élément élastique allongé (10),
- une tête applicatrice (26) pour appliquer sur ledit matériau en nappe (W) ledit au moins un élément élastique allongé (10), et
- une structure-support (20, 22, 24) pour déplacer ladite tête applicatrice (26) sélectivement dans une direction transversale (x) par rapport à ladite direction d'avance (z) dudit matériau en nappe (W), en donnant audit matériau élastique (10) appliqué sur ledit matériau en nappe (W) une trajectoire avec des boucles, où ladite structure-support (20, 22, 24) comprend un élément coulissant (22) portant ladite tête applicatrice (26), ledit élément coulissant (22) étant mobile dans la direction transversale (x) par rapport à ladite direction d'avance (z) et portant ladite tête applicatrice (26) au moyen d'un bras (24) faisant saillie à partir dudit élément coulissant (22) et portant à son extrémité distale ladite tête applicatrice (26),
**caractérisé en ce que** ladite tête applicatrice (26) est une tête tournante avec une configuration en forme de main avec une partie carpienne (26a) et une pluralité de doigts (26b) portant des ouvertures de guidage respectives (26c) pour lesdites formations élastiques comprenant ledit au moins un élément élastique allongé (10), où ladite tête applicatrice (26) peut tourner autour d'un axe (y) substantiellement orthogonal à ladite direction d'avance (z) dudit matériau en nappe (W) et à ladite direction transversale (x) par rapport à ladite direction d'avance (z) dudit matériau en nappe (W) de manière à suivre le mouvement d'orientation dudit au moins un élément allongé élastique (10) durant son application sur ledit matériau en nappe.

2. Le dispositif selon la revendication 1, **caractérisé en ce qu'**il comprend un élément applicateur (32) pour appliquer sur ledit matériau en nappe (W), en correspondance avec ladite tête applicatrice (26), un autre matériau en nappe (W1) pour enfermer, conformément à une configuration générale en sandwich, ledit au moins un élément élastique allongé (10) entre ledit matériau en nappe (W) et ledit autre matériau en nappe (W1).

3. Le dispositif selon l'une ou l'autre des revendications précédentes 1 ou 2, **caractérisé en ce qu'**il comprend des éléments de contrôle de la délivrance (12, 14) dudit au moins un élément élastique allongé (10) dans la partie comprise entre ladite source d'alimentation (12) et ladite tête applicatrice (26).

4. Le dispositif selon l'une des revendications précédentes 1 à 3, **caractérisé en ce que** ladite tête applicatrice (26) comprend une série d'ouvertures de guidage (26c) pour guider une série respective de formations élastiques constituant ledit au moins un élément élastique allongé (10).

5. Le dispositif selon l'une des revendications précédentes 1 à 4, **caractérisé en ce qu'**il comprend :
- au moins deux desdites sources (12) délivrant des éléments élastiques allongés (10),
- au moins deux dites têtes applicatrices (26) pour appliquer sur ledit matériau en nappe (W) au moins deux éléments élastiques allongés (10).
